# EUROPÄISCHE PATENTANMELDUNG

(11) **EP 0 731 087 A1**
(43) Veröffentlichungstag der Anmeldung: **11.09.1996**
(21) Anmeldenummer: 96102713.3
(22) Anmeldetag: 23.02.1996
(51) Int. Cl.: C07C 253/28

(54) **Verfahren zur Herstellung von 2,4-Dichlor-5-fluorbenzonitril und 2,6-Dichlor-3-fluorbenzonitril**

(30) Priorität: 07.03.1995 DE 19507912
(71) Anmelder: BAYER AG, 51368 Leverkusen (DE)
(72) Erfinder: Hagedorn, Ferdinand, Dr., 51381 Leverkusen (DE); Fiege, Helmut, Dr., 51373 Leverkusen (DE); Lantzsch, Reinhard, Dr., 42115 Wuppertal (DE)

(57) **Zusammenfassung**

Ein Gemisch von 2,4-Dichlor-5-fluortoluol und 2,6-Dichlor-3-fluortoluol, welches in wirtschaftlich sinnvoller Weise nicht auftrennbar und einzeln zu den korrespondierenden Benzonitrilen umgesetzt werden kann, wird erfindungsgemäß in Form dieses Gemisches der Ammonoxidation unterworfen und dann auf der Stufe der Benzonitrile durch dem Fachmann bekannte Methoden getrennt.

## Beschreibung

Die vorliegende Erfindung betrifft ein Verfahren zur Herstellung von 2,4-Dichlor-5-fluorbenzonitril und 2,6-Dichlor-3-fluorbenzonitril, bei dem ein Gemisch von 2,4-Dichlor-5-fluortoluol und 2,6-Dichlor-3-fluortoluol durch gemeinsame Ammonoxidation in das Gemisch der genannten Nitrile umgewandelt und dieses anschließend durch dem Fachmann bekannte Methoden in die reinen Nitrile getrennt wird.

Die genannten Nitrile sind wertvolle Zwischenprodukte für Pharmazeutika und Pflanzenschutzwirkstoffe (EP 609 734).

Für die Herstellung von 2,4-Dichlor-5-fluorbenzonitril sind bereits verschiedene Synthesewege beschrieben worden. So ist die Umsetzung von 2,4-Dichlor-5-fluorbenzoesäure mit Thionylchlorid in Gegenwart von katalytischen Mengen Dimethylformamid bekannt geworden; die weitere Umsetzung des erhaltenen Säurechlorids mit Ammoniak zum entsprechenden Säureamid und dessen Dehydratisierung mit Phosphoroxychlorid ergibt sodann das zugehörige Benzonitril (EP 433 124). Ferner ist die Bromierung von 1,3-Dichlor-4-fluorbenzol zu 1-Brom-2,4-dichlor-5-fluorbenzol und dessen weitere Umsetzung mit Kupfer(I)-cyanid zum gewünschten Nitril bekannt (EP 500 083). Einen weiteren Syntheseweg, bei dem 2,4-Dichlor-5-fluornitrobenzol zur Aminoverbindung reduziert und diese nach Diazotierung und Sandmeyer-Reaktion mit Cyanid zum Nitril umgesetzt wird, beschreibt CN 1.031.074 (zitiert nach C.A. 113 (1990), 77918q). Schließlich beschreibt EP 431 373 eine Synthese, bei der 2,4-Dichlor-fluorbenzol mit Tetrachlorkohlenstoff trichlormethyliert und das 50 erhaltene substituierte Benzotrichlorid mit wäßrigem Ammoniak zum Nitril umgesetzt wird.

Alle vorgenannten Synthesemethoden sind mit zum Teil gravierenden Nachteilen behaftet, wobei z.B. teure Ausgangsmaterialien, die Handhabung von Brom, Kupfercyanid oder Tetrachlorkohlenstoff oder eine Vielzahl von Reaktionsstufen oder das Arbeiten in verdünnten Lösungen den Zugang zum gewünschten Nitril erschweren, bzw. das jeweilige Herstellverfahren unwirtschaftlich machen.

Für 2,6-Dichlor-3-fluorbenzonitril ist bisher nur ein sehr aufwendiges Syntheseverfahren beschrieben worden, bei dem 3-Chlor-2,4-difluor-nitrobenzol durch Austausch des in Position 2 befindlichen Fluoratoms mit Cyanid in Dimethylformamid als Reaktionsmedium zum 2-Chlor-3-fluor-6-nitro-benzonitril umgesetzt und durch Chlorolyse der Nitrogruppe bei 190°C in das gewünschte Nitril übergeführt wird. Insbesondere der letzte der genannten Reaktionsschritte ist wegen der hohen Materialbelastung der benutzten Apparate als technisch wenig aussichtsreich anzusehen (JP 03/90057 (1991); zitiert nach C.A. 115 (1991), 182866k).

Aufgabe der vorliegenden Erfindung war es daher, ein einfaches, technisch und wirtschaftlich vorteilhaftes Herstellverfahren für die beiden isomeren Nitrile bereitzustellen.

Ein nach DE-OS 4 340 854 durch zweifache Kernchlorierung von m-Fluortoluol leicht herstellbares Isomerengemisch von 2,4-Dichlor-5-fluortoluol und 2,6-Dichlor-3-fluortoluol kann durch fraktionierende Destillation wegen der zu geringen Siedepunktdifferenz praktisch nicht aufgetrennt werden. Es wurde jetzt gefunden, daß man durch Ammonoxidation des genannten Dichlor-fluortoluol-Gemisches ein Gemisch der gewünschten Dichlor-fluorbenzonitrile erhält und daß man dieses Gemisch der Nitrile durch dem Fachmann bekannte Methoden auftrennen und so die gewünschten Nitrile einzeln isolieren kann.

Die Erfindung betrifft ein Verfahren zur Herstellung von 2,4-Dichlor-5-fluorbenzonitril und 2,6-Dichlor-3-fluorbenzonitril, das dadurch gekennzeichnet ist, daß man ein Gemisch aus 2,4-Dichlor-5-fluortoluol und 2,6-Dichlor-3-fluortoluol mit Ammoniak, Luft und Wasserdampf in einem Molverhältnis von Dichlor-fluortoluol : Ammoniak : Luft : Wasserdampf von 1 : 1-3 : 7,5-15 : 0-10 in der Gasphase bei einer Temperatur von 350 bis 550°C an einem Ammonoxidationskatalysator zu einem Gemisch der genannten Nitrile umsetzt und dieses anschließend durch dem Fachmann bekannte Methoden trennt.

Das Gemisch der genannten Dichlor-fluortoluole kann erfindungsgemäß bei beliebigen Isomerenverhaltnissen zur Ammonoxidation eingesetzt werden. In bevorzugter Weise werden Gemische mit einem Gehalt von 60 bis 90 % an 2,4-Dichlor-5-fluortoluol und 40 bis 10 % an 2,6-Dichlor-3-fluortoluol eingesetzt.

In weiterhin bevorzugter Weise wird die Ammonoxidation in Gegenwart von Wasserdampf gearbeitet; das Molverhältnis der Eingangsstoffe beträgt dann Dichlor-fluortoluol : Ammoniak : Luft : Wasserdampf = 1 : 1,1-1,5 : 7,5-15 : 5-10.

Die für die Ammonoxidation einsetzbaren Katalysatoren sind dem Fachmann bekannt. Hierzu können beispielsweise Verbindungen des Molybdäns, Bismuts, Vanadiums, Chroms, gegebenenfalls mit weiteren Zusätzen auf aktivierten sauren Trägersubstanzen, wie etwa Borphosphat und/oder Zinnphosphat und/oder Kieselsäure und/oder Aluminiumoxid eingesetzt werden (DE-PS 1 189 976; DE-PS 1 770 841). Weitere geeignete Katalysatoren enthalten Vanadiumphosphat (Dissertation F.G. Martin, Erlangen 1989; DD 256 129). Solche und ähnliche Ammonoxidationskatalysatoren sind beispielsweise bereits zur Umsetzung anderer kernhalogenierter Toluole, wie Chlortoluole und Dichlortoluole, eingesetzt worden.

Die Temperatur für die Ammonoxidation liegt im Bereich von 350 bis 550°C, bevorzugt von 400 bis 530°C. Sie ist u.a. abhängig von der Natur des gewählten Katalysators: So erfordern etwa mit MoBi-aktivierte Kontakte tendenziell eine etwas höhere Temperatur im genannten Bereich, während V-haltige Katalysatoren bereits im unteren Teil des genannten Temperaturbereichs leistungsfähig sind.

Das bei der Ammonoxidation anfallende Produktgemisch setzt sich im wesentlichen aus den beiden Nitrilen, den nicht vollständig umgesetzten Dichlor-fluortoluolen und Reaktionswasser zusammen. Hinzu tritt nicht verbrauchter Ammoniak und nicht verbrauchter Luftsauerstoff. Die organischen Wertstoffe, nämlich die Dichlor-fluorbenzonitrile und die Dichlor-fluortoluole können durch Wasserdampfdestillation leicht von etwaigen Verunreinigungen abgetrennt und nach dem Entfernen des Wassers einer Feindestillation zugeführt werden. Hierbei wird zunächst der Anteil der Dichlor-fluortoluole abdestilliert und kann in die Reaktion zurückgeführt werden.

Die weitere Trennung der zurückbleibenden beiden Nitrile gelingt durch dem Fachmann bekannte Methoden, wie durch fraktionierende Destillation, Lösungskristallisation oder Schmelzkristallisation sowie präparative Chromatographie. In bevorzugter Weise wird die fraktionierende Destillation oder die Kristallisation als eine solche Trennmethode eingesetzt. Für den Fall der Benutzung einer fraktionierenden Destillation wird diese bevorzugt im Vakuum durchgeführt. So erhält man beispielsweise unter einem Druck von 140 mbar das 2,4-Dichlor-5-fluorbenzonitril bei einein Siedepunkt von 170,3°C, während das 2,6-Dichlor-3-fluorbenzonitril unter dem gleichen Druck bei 182,2°C siedet. Auf diese Art können beide Nitrile mit einer Reinheit von mehr als 99 % isoliert werden. Es ist gleichermaßen möglich, das aus der Ammonoxidation erhaltene Gemisch ohne Abtrennung der nicht umgesetzten Dichlor-fluortoluole mit Impfkristallen eines der gewünschten Dichlor-fluorbenzonitrile zu versetzen und das hierbei erhältliche Kristallisat des betreffenden Dichlor-fluorbenzonitrils durch Filtration zu gewinnen; in bevorzugter Weise wird das Animpfen zur Gewinnung des 2,4-Dichlor-5-fluorbenzonitrils durchgeführt.

### Beispiel 1

Über 170 ml eines Katalysator-Granulats mit einem Korndurchmesser von 0,25 mm, bestehend aus mit 3,5 % Molybdänoxid und 4,5 % Bismutoxid aktiviertem Borphosphat-Zinnphosphat-Kieselsäure-Trägermaterial, angeordnet in einem Reaktionsrohr, wurde bei Temperaturen zwischen 500 und 530°C ein Gasgemisch erzeugt aus Dichlor-fluortoluol, Ammoniak, Luft und Wasser im Molverhältnis von ca. 1 : 3 : 13,5 : 10 geleitet. Pro Minute wurden im einzelnen 1,1 g Dichlor-fluortoluol (d = 1,36 g/ml), 0,26 g Ammoniak, 2 l Luft und 1,1 g Wasser durchgesetzt. Bei den angegebenen Temperaturen erfolgte die Reaktion in der Wirbelschicht. Die das Reaktionsrohr verlassenden Dämpfe wurden kondensiert und mit Methanol und Wasser gewaschen. Die erhaltene Lösung wurde auf ihren Gehalt an Dichlor-fluortoluol und Dichlor-fluorbenzonitrilen gaschromatographisch analysiert.

Bei einer Reaktionstemperatur von 520°C betrug die Ausbeute, bezogen auf zu 55 % umgesetztes Dichlor-fluortoluol (= Selektivität), 81 bis 85 % der theoretischen Ausbeute (Ergebnisse mehrerer Reaktionsläufe).

### Beispiel 2

Die Ammonoxidation eines Dichlor-fluortoluol-Gemisches wurde gemäß den Angaben des Beispiels 1 wiederholt, wobei das den Reaktor verlassende Reaktionsgemisch mit Toluol und Wasser gewaschen wurde. Die Toluolphase wurde nach Abtrennen der Wasserphase über eine 40 cm lange, mit Glasringen (⌀ 4 mm) gefüllte Silberspiegel-Kolonne destilliert. Nach Abnahme des Toluols erhielt man bei einer Kopftemperatur von 41°C, einem Vakuum von 1,2 bis 1,4 mbar und einem Rücklaufverhältnis von 1 : 1 das Gemisch der nicht umgesetzten Dichlorfluortoluole. Das zurückbleibende Gemisch der Nitrile wurde über eine Kolonne mit verbesserter Trennwirkung (ca. 40 theoretische Böden) im Vakuum bei einem Rücklaufverhältnis 10 : 1 fraktionierend destilliert. Bei 140 mbar wurde beim Siedepunkt 170,3°C ein 99,7%iges 2,4-Dichlor-5-fluorbenzonitril und beim Siedepunkt 182,2°C ein 99,2%iges 2,6-Dichlor-3-fluorbenzonitril gewonnen.

### Beispiel 3

Ein flüssiges Gemisch der Zusammensetzung 23,7 % 2,4-Dichlor-5-fluortoluol, 11,4 % 2,6-Dichlor-3-fluortoluol, 57,9 % 2,4-Dichlor-5-fluorbenzonitril und 4,3 % 2,6-Dichlor-3-fluorbenzonitril wurde bei Raumtemperatur mit Kristallen von > 99 %igem 2,4-Dichlor-5-fluorbenzonitril geimpft. Nach kurzer Zeit bildeten sich reichlich derbe Kristalle, die sich nach dem Absaugen ohne weitere Wäsche mit einem Lösungsmittel als 97%iges 2,4-Dichlor-5-fluorbenzonitril (GC) erwiesen; als Verunreinigung enthielten sie noch 1,7 % 2,4-Dichlor-5-fluortoluol und nur 0,2 % 2,6-Dichlor-3-fluorbenzonitril (Rest nicht identifiziert).

### Beispiel 4

Ein Katalysatorgranulat mit einem Korndurchmesser von 0,25 mm, bestehend aus einem Gemisch von 20 % Vanadylphosphat, 20 % Borphosphat, 20 % Zinn(IV)-pyrophosphat und 40 % SiO₂ wurde mit einem Gasgemisch, wie im Beipsiel 1 angegeben, bei 470°C durchströmt. Die Reaktionsgase wurden nach Verlassen des Reaktionsrohres mit Methanol und Wasser gewaschen, und die erhaltene Lösung wurde analysiert (GC). Bei einem Umsatz von 70 % des Dichlor-fluortoluol-Gemisches wurde, bezogen auf dessen Umsatz, eine Ausbeute von 75 % (= Selektivität) Dichlor-fluorbenzonitril-Gemisch festgestellt.

## Patentansprüche

1. Verfahren zur Herstellung von 2,4-Dichlor-5-fluorbenzonitril und 2,6-Dichlor-3-fluorbenzonitril, dadurch gekennzeichnet, daß man ein Gemisch aus 2,4-Dichlor-5-fluortoluol und 2,6-Dichlor-3-fluortoluol mit Ammoniak, Luft und Wasserdampf in einem Molverhältnis von Dichlor-fluortoluol : Ammoniak : Luft : Wasserdampf = 1 : 1-3 : 7,5-15 : 0-10 in der Gasphase bei einer Temperatur von 350 bis 550°C an einem Ammonoxidationskatalysator zu einem Gemisch der genannten Nitrile umsetzt und dieses Gemisch anschließend durch dem Fachmann bekannte Methoden trennt.

2. Verfahren gemäß Anspruch 1, dadurch gekennzeichnet, daß man ein Gemisch aus 60 bis 90 % an 2,4-Dichlor-5-fluortoluol und 40 bis 10 % 2,6-Dichlor-3-fluortoluol einsetzt.

3. Verfahren gemäß Anspruch 1, dadurch gekennzeichnet, daß man ein Dichlor-fluortoluol-Isomerengemisch einsetzt, wie es bei der zweifachen Kernchlorierung von m-Fluortoluol anfällt.

4. Verfahren nach Anspruch 1, dadurch gekennzeichnet, daß man die Ammonoxidation im Temperaturbereich von 400 bis 530°C durchführt.

5. Verfahren nach Anspruch 1, dadurch gekennzeichnet, daß man bei einem Molverhältnis Dichlor-fluortoluol : Ammoniak : Luft : Wasserdampf von 1 : 1,1-1,5 : 7,5-15 : 5-10 arbeitet.

6. Verfahren nach Anspruch 1, dadurch gekennzeichnet, daß man das Gemisch der beiden Nitrile durch Destillation oder Kristallisation trennt.

7. Verfahren nach Anspruch 6, dadurch gekennzeichnet, daß man im Reaktionsgemisch, das nicht umgesetzte Dichlor-fluortoluole und die genannten Dichlor-fluorbenzonitrile enthält, das 2,4-Dichlor-5-fluorbenzonitril zur Kristallisation bringt und als Kristallisat abtrennt.
